# EUROPÄISCHE PATENTANMELDUNG

(11) **EP 3 984 505 A1**
(43) Veröffentlichungstag der Anmeldung: **20.04.2022**
(21) Anmeldenummer: 21165417.3
(22) Anmeldetag: 27.03.2021
(51) Int. Cl.: A61F 13/49, B32B 3/28, B32B 5/02, B32B 5/04, B32B 7/04, B32B 25/10, B32B 27/12, B32B 27/32

(54) **BAHNENFÖRMIGES VLIESSTOFFLAMINAT**

(30) Priorität: 13.10.2020 DE 102020126818
(71) Anmelder: K.L. Kaschier- und Laminier GmbH, 48455 Bad Bentheim-Gildehaus (DE)
(72) Erfinder: BALDAUF, Georg, 48366 Laer (DE); SCHULZE WEHNINCK, Rembert, 82327 Tutzing (DE)
(74) Vertreter: Cohausz Hannig Borkowski Wißgott

(57) **Zusammenfassung**

Die Erfindung betrifft eine Verfahren zum Herstellen eines bahnenförmigen Vliesstofflaminats mit zwei Vliesstoffen und einer dazwischen liegenden, elastischen Folie, wobei die drei Laminatschichten an zahlreichen Schweißpunkten miteinander verschweißt werden, wobei die elastische Folie vor dem Verschweißen quer zur Bahnlaufrichtung um das 1,5 bis 3-fache gedehnt wird, wobei das Verschweißen der drei Schichten bei gedehnter Folie mit Schweißpunktreihen erfolgt, die wellenförmig sind und quer zur Bahnlaufrichtung verlaufen, so dass nach dem Verschweißen der drei Schichten und dem Entspannen der Folie beide Vliesstoffe in Falten quer zur Bahnlaufrichtung liegen und die Schweißpunktreihen wellenförmig sind mit kürzerer Wellenlänge als bei gedehnter Folie.

## Beschreibung

Die Erfindung betrifft ein bahnenförmiges Vliesstofflaminat mit zwei Vliesstoffen und einer dazwischen liegenden, elastischen Folie, wobei die drei Laminatschichten an zahlreichen Schweißpunkten miteinander verschweißt werden.

Es ist bekannt, dass bei Produkten, die Vliesstoffe verwenden insbesondere für Hygieneprodukte, eine angenehme Haptik insbesondere eine hohe Weichheit von Bedeutung ist. Hierbei sind aber durch die verwendeten Materialien Grenzen gesetzt.

Aufgabe der Erfindung ist es, bei einfachem Herstellungsverfahren ein bahnenförmiges Vliesstofflaminat insbesondere für Hygieneprodukte zu schaffen, das in dem Endprodukt den Eindruck von "Weichheit" und "Drapierfähigkeit" vermittelt.

Diese Aufgabe wird erfindungsgemäß dadurch gelöst,
- dass die elastische Folie vor dem Verschweißen quer zur Bahnlaufrichtung um das 1,5 bis 3-fache gedehnt wird,
- dass das Verschweißen der drei Schichten bei gedehnter Folie mit Schweißpunktreihen erfolgt, die wellenförmig sind und quer zur Bahnlaufrichtung verlaufen,
- so dass nach dem Verschweißen der drei Schichten und dem Entspannen der Folie beide Vliesstoffe in Falten quer zur Bahnlaufrichtung liegen und die Schweißpunktreihen wellenförmig sind mit kürzerer Wellenlänge als bei gedehnter Folie.

Durch ein solches Verfahren wird nach der Relaxierung eine deutlich sichtbare wellenförmige, visuelle Struktur der Schweißpunktlinien geschaffen, die den Anschein ergibt, dass ein Endprodukt insbesondere ein Hygieneprodukt in dem Bereich, in dem ein solches Vliesstofflaminat verwendet wird, eine hohe Weichheit und eine optimale Drapierfähigkeit aufweist. Hierbei zeichnet sich das Verfahren durch einfache Herstellungsschritte und durch eine einfache Weiterverarbeitung zum Endprodukt aus.

Vorteilhafte Ausgestaltungen des erfindungsgemäßen Verfahrens sind in den Unteransprüchen aufgeführt.

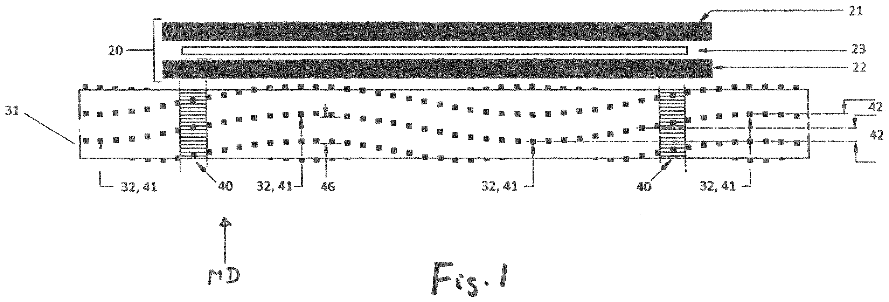

Ausführungsbeispiele der Erfindung werden im Folgenden näher beschrieben. Die schematischen Zeichnungen zeigen in Ausschnitten in
- Fig. 1: einen Querschnitt durch das erfindungsgemäße bahnenförmige Vliesstofflaminat quer zur Bahnlaufrichtung mit unter dem Laminat angeordneter Gravurwalze,
- Fig. 2: einen Ausschnitt aus einer Draufsicht auf die Gravurwalze und

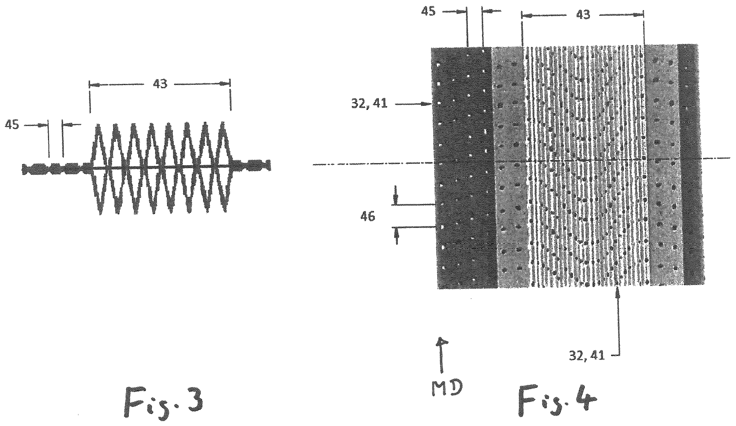

- Fig. 3: einen Querschnitt durch das Vliesstofflaminat nach dem Entspannen der elastischen Folie mit schematisch dargestellten Falten der Vliesstoffe.
- Fig. 4: eine Draufsicht auf das Vliesstofflaminat nach dem Entspannen und befestigt am Rand einer Windel.

Zum Herstellen des bahnenförmigen Vliesstofflaminats 20 wird zwischen zwei Vliesstoffen 21, 22 aus Polymerfasern vorzugsweise aus Polyolefinfasern eine elastische Folie 23 aus SIS oder SEBs gelegt, damit diese drei Kunststoffschichten miteinander verschweißt werden. Vor dem Verschweißen wird die mittig liegende elastische Folie 23 quer zur Bahnlaufrichtung MD um das 1,5 bis 3-fache vorzugsweise um das 2,6 bis 2,8-fache gedehnt. Die drei Schichten werden bei gedehnter mittiger Folie 23 auf eine Schweißwalze/Gravurwalze 31 gelegt, die die Schichten durch Vakuum 40 festhält.

Die Walze 31 weist erhabene Gravurpunkte/Schweißpunkte 32 auf, die aufgrund ihrer Ultraschall-Erwärmung die drei Schichten an diesen Stellen aneinander schweißt. Hierbei wird die elastische Folie 23 durchschmolzen. Die Schweißpunkte 32 sind in Schweißpunktreihen angeordnet, die quer zur Bahnlaufrichtung MD liegen.

Die Schweißpunkte 32 der Schweißwalze bilden in den Schweißpunkreihen sinusförmige Wellen mit einer Amplitude 42, die das 0,2 bis 0,5-fache des Abstands 46 der Schweißpunktreihen voneinander in Bahnlaufrichtung MD beträgt. Hierbei beträgt der Abstand 46 der Schweißpunktreihen voneinander in Bahnlaufrichtung MD 2 bis 20 mm vorzugsweise 3 bis 5 mm. Die Schweißpunkte 32 weisen auf der Schweißwalze 31 innerhalb einer Schweißpunktreihe voneinander einen Abstand 45 von 2 bis 4 mm, vorzugsweise von 2,2 bis 2,6 mm auf.

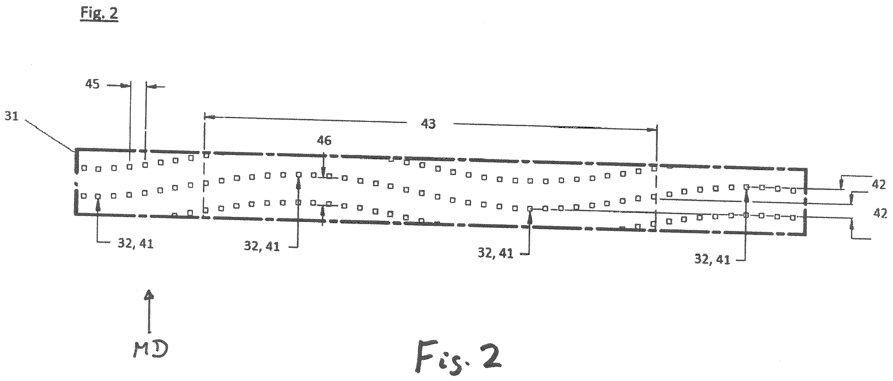

Die Schweißwalze 31 weist Vakuumöffnungen 40 auf, die die endseitigen Fixierstellen der elastischen Folie 23 im gedehnten Zustand festhalten. In Fig. 2 ist die Wellenlänge 43 der Schweißpunktreihen dargestellt. Sobald das Laminat von der Schweißwalze genommen wird, zieht sich die elastische Folie 23 zusammen und erzeugt aufgrund der dann geringeren Wellenlänge Falten in beiden Vliesstoffen 21, 22, die zueinander parallel und quer zur Bahnlaufrichtung MD liegen. Aufgrund dieser Falten ist das Laminat dehnfähig, um z. B. in Hygieneprodukten verwendet zu werden, insbesondere als elastischer Windelverschlussträger in Babywindeln.

Im ungedehnten, relaxierten Zustand ist die Wellenform aufgrund der geringeren Wellenlänge besonders gut sichtbar, d. h. die wellenförmige Struktur kommt beim Produkt optimal zur Geltung und symbolisiert eine hohe Weichheit und Drapierfähigkeit.

Die Vliesstoffe 21, 22 weisen ein Gewicht von 10 bis 30 g/m² und die elastische Folie 23 ein Gewicht von 30 bis 110 g/m² auf. Die Folie 23 nimmt 25 bis 100 Prozent der gesamten Fläche des Laminats ein.

## Patentansprüche

1. Verfahren zum Herstellen eines bahnenförmigen Vliesstofflaminats mit zwei Vliesstoffen (21, 22) und einer dazwischen liegenden, elastischen Folie (23), wobei die drei Laminatschichten an zahlreichen Schweißpunkten (32) miteinander verschweißt werden, **dadurch gekennzeichnet,**
- **dass** die elastische Folie (23) vor dem Verschweißen quer zur Bahnlaufrichtung (MD) um das 1,5 bis 3-fache gedehnt wird,
- **dass** das Verschweißen der drei Schichten bei gedehnter Folie mit Schweißpunktreihen erfolgt, die wellenförmig sind und quer zur Bahnlaufrichtung verlaufen,
- so dass nach dem Verschweißen der drei Schichten und dem Entspannen der Folie (23) beide Vliesstoffe (21, 22) in Falten quer zur Bahnlaufrichtung (MD) liegen und die Schweißpunktreihen wellenförmig sind mit kürzerer Wellenlänge (43) als bei gedehnter Folie.

2. Vliesstofflaminat nach Anspruch 1, **dadurch gekennzeichnet, dass** die elastische Folie (23) um das 2,6 bis 2,8-fache gedehnt wird.

3. Verfahren nach Anspruch 1 oder 2, **dadurch gekennzeichnet, dass** die elastische Folie (23) im gedehnten Zustand auf einer Schweißwalze (31) mittels Vakuum gehalten und durch Ultraschall mit den Vliesstoffen verschweißt wird.

4. Verfahren nach einem der vorherigen Ansprüche, **dadurch gekennzeichnet, dass** die Schweißpunkte (32) auf der Schweißwalze (31) innerhalb einer Schweißpunktreihe (41) voneinander einen Abstand (45) von 2 bis 4 mm, vorzugsweise von 2,2 bis 2,6 mm aufweisen.

5. Verfahren nach einem der vorherigen Ansprüche, **dadurch gekennzeichnet, dass** der Abstand (46) der Schweißpunktreihen voneinander in Bahnlaufrichtung (MD) 2 bis 20 mm vorzugsweise 3 bis 5 mm beträgt.

6. Verfahren nach einem der vorherigen Ansprüche, **dadurch gekennzeichnet, dass** die Amplitude (42) der Schweißpunktreihen (Schweißpunktwellen) das 0,2 bis 0,5-fache des Abstands (46) der Schweißpunktreihen voneinander in Bahnlaufrichtung (MD) beträgt.

7. Verfahren nach einem der vorherigen Ansprüche, **dadurch gekennzeichnet, dass** die Falten der Vliesstoffe (21, 22) zueinander parallel und quer zur Bahnlaufrichtung (MD) liegen.

8. Verfahren nach einem der vorherigen Ansprüche, **dadurch gekennzeichnet, dass** die Vliesstoffe (21, 22) ein Gewicht von 10 bis 30 g/m² aufweisen.

9. Verfahren nach einem der vorherigen Ansprüche, **dadurch gekennzeichnet, dass** die elastische Folie (23) ein Gewicht von 30 bis 110 g/m² aufweist.

10. Verfahren nach einem der vorherigen Ansprüche, **dadurch gekennzeichnet , dass** die Folie (23) 25 bis 100 Prozent der gesamten Fläche des Laminats einnimmt.

## Geänderte Patentansprüche

### Geänderte Patentansprüche gemäss Regel 137(2) EPÜ.

1. Verfahren zum Herstellen eines bahnenförmigen Vliesstofflaminats mit zwei Vliesstoffen (21, 22) und einer dazwischen liegenden, elastischen Folie (23), wobei die drei Laminatschichten an zahlreichen Schweißpunkten (32) miteinander verschweißt werden, wobei die elastische Folie (23) vor dem Verschweißen quer zur Bahnlaufrichtung (MD) um das 1,5 bis 3-fache gedehnt wird, das Verschweißen der drei Schichten bei gedehnter Folie mit Schweißpunktreihen erfolgt, die wellenförmig sind und quer zur Bahnlaufrichtung verlaufen, und nach dem Verschweißen der drei Schichten und dem Entspannen der Folie (23) beide Vliesstoffe (21, 22) in Falten quer zur Bahnlaufrichtung (MD) liegen und die Schweißpunktreihen wellenförmig sind mit kürzerer Wellenlänge (43) als bei gedehnter Folie, **dadurch gekennzeichnet, dass** die elastische Folie (23) im gedehnten Zustand auf einer Schweißwalze (31) mittels Vakuum gehalten und durch Ultraschall mit den Vliesstoffen verschweißt wird.

2. Vliesstofflaminat nach Anspruch 1, **dadurch gekennzeichnet, dass** die elastische Folie (23) um das 2,6 bis 2,8-fache gedehnt wird.

3. Verfahren nach Anspruch 1 oder 2, **dadurch gekennzeichnet, dass** die Schweißpunkte (32) auf der Schweißwalze (31) innerhalb einer Schweißpunktreihe (41) voneinander einen Abstand (45) von 2 bis 4 mm, vorzugsweise von 2,2 bis 2,6 mm aufweisen.

4. Verfahren nach einem der vorherigen Ansprüche, **dadurch gekennzeichnet, dass** der Abstand (46) der Schweißpunktreihen voneinander in Bahnlaufrichtung (MD) 2 bis 20 mm vorzugsweise 3 bis 5 mm beträgt.

5. Verfahren nach einem der vorherigen Ansprüche, **dadurch gekennzeichnet, dass** die Amplitude (42) der Schweißpunktreihen (Schweißpunktwellen) das 0,2 bis 0,5-fache des Abstands (46) der Schweißpunktreihen voneinander in Bahnlaufrichtung (MD) beträgt.

6. Verfahren nach einem der vorherigen Ansprüche, **dadurch gekennzeichnet, dass** die Falten der Vliesstoffe (21, 22) zueinander parallel und quer zur Bahnlaufrichtung (MD) liegen.

7. Verfahren nach einem der vorherigen Ansprüche, **dadurch gekennzeichnet, dass** die Vliesstoffe (21, 22) ein Gewicht von 10 bis 30 g/m² aufweisen.

8. Verfahren nach einem der vorherigen Ansprüche, **dadurch gekennzeichnet, dass** die elastische Folie (23) ein Gewicht von 30 bis 110 g/m² aufweist.

9. Verfahren nach einem der vorherigen Ansprüche, **dadurch gekennzeichnet, dass** die Folie (23) 25 bis 100 Prozent der gesamten Fläche des Laminats einnimmt.
